Europäisches Patentamt

European Patent Office

Office européen des brevets

(19)

(11) Veröffentlichungsnummer: **0 056 158**
**A1**

(12) **EUROPÄISCHE PATENTANMELDUNG**

(21) Anmeldenummer: 81110812.5

(22) Anmeldetag: 29.12.81

(51) Int. Cl.³: **C 08 G 18/02,** C 07 D 251/34,
C 08 G 18/22, C 07 D 323/00,
C 08 G 18/79, C 08 G 18/80

(30) Priorität: 08.01.81 DE 3100262

(43) Veröffentlichungstag der Anmeldung: 21.07.82
Patentblatt 82/29

(84) Benannte Vertragsstaaten: BE DE FR GB IT NL

(71) Anmelder: BAYER AG, Zentralbereich Patente, Marken
und Lizenzen, D-5090 Leverkusen 1, Bayerwerk (DE)

(72) Erfinder: Richter, Roland, Dr., Burgstrasse 43,
D-5090 Leverkusen 1 (DE)
Erfinder: Müller, Hanns Peter, Dr., Im Kerberich 6,
D-5068 Odenthal (DE)
Erfinder: Wagner, Kuno, Dr., Am Kiesberg 8,
D-5090 Leverkusen (DE)

(54) Verfahren zur Herstellung von Isocyanuratgruppen aufweisenden Polyisocyanaten, als Katalysator-Komponente für dieses Verfahren geeignete Lösungen, sowie die Verwendung der Verfahrensprodukte als Isocyanat-Komponente bei der Herstellung von Polyurethanen.

(57) Verfahren zur Herstellung von Isocyanuratgruppen aufweisenden Polyisocyanaten durch katalytische Trimerisierung eines Teils der Isocyanatgruppen von organischen Polyisocyanaten oder von Gemischen aus Di- und Monoisocyanaten und Abbruch der Trimerisierungsreaktion durch Zugabe eines Katalysatorengifts, wobei als Trimerisierungskatalysatoren 1:1-Komplexe von (i) basischen Natrium- oder Kaliumverbindungen und (ii) 1,4,7,10,13-Pentaoxacyclopentadecan bzw. 1,4,7,10,13,16-Hexaoxacyclooctadecan verwendet werden, zur Durchführung des Verfahrens als Katalysatorkomponente geeignete Lösungen derartiger 1:1-Komplexe in polaren Lacklösungsmitteln und/oder alkoholische Hydroxylgruppen aufweisenden Verbindungen des Molekulargewichtsbereichs 32–250, sowie die Verwendung der Verfahrensprodukte als Isocyanatkomponente bei der Herstellung von Polyurethanen.

EP 0 056 158 A1

- 1 -

0056158

BAYER AKTIENGESELLSCHAFT    5090 Leverkusen, Bayerwerk

Zentralbereich                              Wr/m-c
Patente, Marken und Lizenzen

Verfahren zur Herstellung von Isocyanuratgruppen
aufweisenden Polyisocyanaten, als Katalysator-Komponente
für dieses Verfahren geeignete Lösungen, sowie die Verwendung der Verfahrensprodukte als Isocyanat-Komponente
bei der Herstellung von Polyurethanen

Die Erfindung betrifft ein neues Verfahren zur Herstellung von Isocyanuratgruppen aufweisenden Polyisocyanaten durch teilweise Trimerisierung der Isocyanatgruppen von organischen Polyisocyanaten, oder von Gemischen aus Di- und Monoisocyanaten, eine zur Durchführung dieses Verfahrens geeignete Katalysatorlösung
sowie die Verwendung der Verfahrensprodukte als Isocyanat-Komponente bei der Herstellung von Polyurethanen nach dem Isocyanat-Polyadditionsverfahren.

Verfahren zur Trimerisierung organischer Isocyanate,
insbesondere Polyisocyanate, sind in großer Zahl bekannt
(J. H. Saunders u. K.C. Frisch, Polyurethanes Chemistry
and Technology, S. 94 ff. (1962). Als Katalysatoren für
die Trimerisierung sind starke organische Basen geeignet,
so z.B. die alkalisch wirkenden Metallsalze von Carbonsäuren, Metallalkoholate, Metallphenolate, Alkalimetallcarbonate, tert. Amine, tert. Phosphine und die "Onium"-
Verbindungen von Stickstoff und Phosphor sowie basische

Le A 20 780

Heterocyclen dieser Elemente. Die Katalysatoren werden dabei häufig in Kombination eingesetzt oder zusammen mit weiteren Co-Katalysatoren wie Mono-N-substituierten Carbaminsäureestern (A. Farkas u. G. A. Mills, Advances in Catalysis, Vol. 13, 393 (1962).

Verfahren zur Herstellung hochwertiger Isocyanuratgruppen aufweisender Polyisocyanate arbeiten meist mit aufwendigen Katalysatorsystemen, da bekannt ist, daß einfache Metallsalze wie Carboxylate oder Alkoholate nur in relativ hoher Konzentration und bei hoher Temperatur eine Cyclotrimerisierung von Isocyanaten bewirken; so benötigt man z.B. zur Trimerisierung von 5 Teilen Phenylisocyanat 1 Teil Kaliumacetat, wobei 3 Stunden auf 100°C erhitzt wird (Houben-Weyl, Bd. 8, S. 244, Thieme V.) (vgl. auch GB-PS 809 809 - Bsp. 6).

Will man die Trimerisierung mit Metallsalzen im Lösungsmittel durchführen, müssen stark polare aprotische Lösungsmittel verwendet werden wie Dimethylformamid oder Dimethylsulfoxid, da nur diese anorganische Metallsalze und Metallsalze mit kleinem organischen Rest zu lösen vermögen (DE-OS 2 839 084). Auch hier sind immer noch Katalysatorkonzentrationen von 0,1 bis 0,5 Gew.-% notwendig. Dies gilt auch, wenn man protische Lösungsmittel verwendet, die aber ihrerseits mit dem Isocyanat Urethane bilden und so die NCO-Zahl senken, bzw. Ausfällungen und Trübungen bilden, so daß das Reaktionsprodukt filtriert werden muß (GB-PS 920 080).

Le A 20 780

Außerdem bewirken die Metallsalze des Standes der Technik nur bei aromatischen Isocyanaten eine schnelle Trimerisierung, aliphatische Mono- und Polyisocyanate benötigen eine hohe Katalysatorkonzentration sowie Temperaturen, die über 50°C liegen, wodurch häufig ein uneinheitlicher exothermer Reaktionsverlauf eintritt und bei Polyisocyanaten hochviskose, stark verfärbte Produkte entstehen (vgl. US-PS 3 330 828, Bsp. 1 - 4; GB-PS 952 931, Bsp. 3; DE-AS 1 013 869, Bsp. 3), oder Gelteilchen gebildet werden (GB-PS 966 338, Bsp. 3), wodurch die Produkte für den Polyurethanlacksektor weitgehend ungeeignet werden. Ein wesentlicher Nachteil bei der Katalyse mit Metallsalzen ist außerdem, daß beim Abstoppen des Katalysators anorganische Salze entstehen, die im Polyisocyanat unlöslich sind und Trübungen verursachen. Die neueren Verfahren des Standes der Technik verwenden deshalb kaum noch die einfachen und billigen basischen Metallsalze wie z.B. Kaliumacetat, sondern je nach Isocyanat werden spezielle organische Basen bei ganz bestimmten Reaktionsbindungen eingesetzt. So nimmt man z.B. zur Trimerisierung von aromatischen Polyisocyanaten Mannich-Basen (DE-OS 2 551 634 und DE-OS 2 641 380) oder tert. Phosphine, wobei zuerst Uretdione gebildet werden, die sich erst in zweiter Reaktionsphase zum Isocyanurat umsetzen (DE-OS 1 201 992). Zur Trimerisierung (cyclo)-aliphatischer Diisocyanate verwendet man häufig organische Basen mit Betain-Struktur wie quarternäre Ammoniumhydroxide (EP-OS 010 589 und EP-OS 009 694), Aminimide (J. E. Kresta, R.J. Chang, S. Kathiriya u. K. C. Frisch, Makromol. Chem. 180, 1081 (1979) sowie Azirinderivate in Kombination mit tert. Aminen (DE-AS 2 325 826).

Le A 20 780

Bei allen diesen Katalysatorsystemen ist nachteilig, daß ganz bestimmte Temperaturintervalle eingehalten werden müssen, teils nur lösungsmittelfrei, teils nur in ausgewählten Lösungsmitteln gearbeitet werden kann und insbesondere entweder nur aromatische oder nur aliphatische Polyisocyanate trimerisiert werden können.

Aufgabe der vorliegenden Erfindung ist es nun, ein Verfahren zur Verfügung zu stellen, nach welchem in technisch einfacher Weise farblose Isocyanuratgruppen aufweisenden Polyisocyanate sowohl aromatischer als auch aliphatischer Struktur im Lösungsmittel oder lösungsmittelfrei und ohne aufwendige Temperaturregelung mit ein und demselben Katalysator hergestellt werden können.

Diese Aufgabe konnte überraschenderweise dadurch gelöst werden, daß man zur Trimerisierung 1:1-Komplexe von basischen Natrium- oder Kaliumverbindungen mit 1,4,7,10,13-Pentaoxacyclopentadecan ("15-Krone-5") bzw. 1,4,7,10,13,16-Hexaoxacyclooctadecan ("18-Krone-6") als Trimerisierungskatalysator verwendet.

Zwar hatte C.J. Pedersen bereits erkannt, daß Kronenether-komplexierte Alkalimetallsalze grundsätzlich als Trimerisierungskatalysatoren für aromatische Isocyanate geeignet sind (J. Am. Chem. Soc. 89, 7017 (1967) oder US-PS 3 686 225), jedoch eignen sich die in diesen Vorveröffentlichungen beschriebenen ankondensierte Benzol- oder Cyclohexanringe aufweisenden Kronenether

Le A 20 780

0056158

bzw. ihre Komplexe mit basischen Natrium- oder Kaliumverbindungen nicht für eine großtechnische Herstellung
von hochwertigen Isocyanuratgruppen aufweisenden Polyisocyanaten, da einerseits die Kronenether mit ankondensierten Cyclohexanringen eine nur äußerst geringe
komplexierende Wirkung für basische Alkalimetallverbindungen aufweisen, und da andererseits 1:1-Komplexe auf
Basis von Kronenethern mit ankondensierten Benzolringen
in organischen Medien sehr schlecht löslich sind, so
daß beispielsweise konzentrierte Katalysatorlösungen
in physiologisch unbedenklichen Lösungsmitteln mit diesen
Komplexen nicht herstellbar sind. Demgegenüber sind die
beim erfindungsgemäßen Verfahren einzusetzenden, nachstehend näher beschriebenen 1:1-Komplexe nicht mit
derartigen Nachteilen behaftet. Die den erfindungswesentlichen 1:1-Komplexen zugrunde liegenden Kronenether
sind einerseits hervorragend zur Herstellung von stabilen Komplexen mit basischen Natrium- und Kaliumverbindungen geeignet, und andererseits weisen diese
Komplexe eine sehr gute Löslichkeit sowohl in den zu
trimerisierenden Polyisocyanaten als auch in den nachstehend näher beschriebenen Hilfslösungsmitteln auf.
Sie können daher in relativ konzentrierter Lösung dem
zu trimerisierenden Polyisocyanat zudosiert werden,
was bei einer großtechnischen Herstellung von Isocyanuratgruppen aufweisenden Polyisocyanaten von Bedeutung
ist.

Gegenstand der Erfindung ist somit ein Verfahren zur
Herstellung von Isocyanuratgruppen aufweisenden Polyisocyanaten durch Trimerisierung eines Teils der

Le A 20 780

Isocyanatgruppen von organischen Polyisocyanaten oder von Gemischen aus Di- und Monoisocyanaten in Gegenwart von basischen Verbindungen als Trimerisierungskatalysatoren und Abbruch der Trimerisierungsreaktion durch Zugabe eines Katalysatorengifts, dadurch gekennzeichnet, daß man als Trimerisierungskatalysatoren 1:1-Komplexe von (i) basischen Natrium- oder Kaliumverbindungen und (ii) 1,4,7,10,13-Pentaoxacyclopentadecan bzw. 1,4,7,10, 13,16-Hexaoxacyclooctadecan verwendet.

Gegenstand der Erfindung sind auch als Katalysator-Komponente für dieses Verfahren geeignete Lösungen von 1:1-Komplexen von (i) basischen Natrium- oder Kaliumverbindungen und (ii) 1,4,7,10,13-Pentaoxacyclopentadecan bzw. 1,4,7,10,13,16-Hexaoxacyclooctadecan in polaren Lacklösungsmitteln und/oder mindestens einer bei Raumtemperatur flüssigen, alkoholische Hydroxylgruppen aufweisenden Verbindung des Molekulargewichtsbereichs 32 - 250.

Gegenstand der Erfindung ist auch die Verwendung der erfindungsgemäßen Verfahrensprodukte, gegebenenfalls in von monomerem Ausgangspolyisocyanat befreiter Form und/oder gegebenenfalls in mit Blockierungsmitteln für Isocyanatgruppen blockierter Form als Isocyanat-Komponente bei der Herstellung von Polyurethanen nach dem Isocyanat-Polyadditionsverfahren.

Im Rahmen der Erfindung sind unter "1:1-Komplexen" Komplexe von äquimolaren Mengen einer basischen Natrium- oder Kaliumverbindung mit 15-Krone-5 bzw. 18-Krone-6

Le A 20 780

zu verstehen. Die Komplexierung der Natriumverbindungen geschieht mit dem erstgenannten, die Komplexierung der Kaliumverbindungen mit dem letztgenannten cyclischen Polyether.

Als basische Natrium- oder Kaliumverbindungen kommen erfindungsgemäß beliebige Verbindungen der genannten Alkali-Metalle in Betracht, deren wäßrige Lösung in einmolarer Konzentration einen pH-Wert von mindestens 7,5 aufweist. Geeignete basische Verbindungen sind beispielsweise Natrium- oder Kaliumcarboxylate mit vorzugsweise 1 bis 12 Kohlenstoffatomen, -alkoholate mit vorzugsweise 1 bis 8 Kohlenstoffatomen, -phenolate mit vorzugsweise 6 bis 10 Kohlenstoffatomen, -carbonate, -hydroxide, -cyanate, Enolate oder -cyanide. Geeignete basische Natrium- oder Kaliumverbindungen sind z.B. die Formiate, Acetate, Propionate, 2-Ethylhexanoate, n-Dodecanoate, Caprylate, Methylate, Ethylate, Butylate, Hexylate, Phenolate, tert.-Butylphenolate, Carbonate, Hydroxide, Cyanate, Thiocyanate oder Cyanide der genannten Metalle oder aber auch beispielsweise Natrium- oder Kalium-N-methylacetamid. Zu den bevorzugten basischen Verbindungen gehören die genannten Carboxylate, Alkoholate, Phenolate, Carbonate, Hydroxide, Cyanate und Cyanide. Besonders bevorzugt sind einfache Carboxylate der genannten Alkali-Metalle mit 1 bis 4 Kohlenstoffatomen, insbesondere Kaliumacetat.

Die zur Komplexbildung eingesetzten cyclischen Polyether stellen bekannte Verbindungen dar. Ihre Herstellung

Le A 20 780

0056158

kann beispielsweise gemäß G. Johns, C.J. Ransom, C.B. Reese, Synthesis (1976), Seite 515 erfolgen.

Die Herstellung der beim erfindungsgemäßen Verfahren einzusetzenden 1:1-Komplexe kann beispielsweise nach einer der nachstehenden Methoden erfolgen:

1. Die Herstellung erfolgt unter Verwendung des zu trimerisierenden Polyisocyanats bzw. seiner Lösung in einem geeigneten Lösungsmittel, welches auch als Reaktionsmedium bei der Durchführung des erfindungsgemäßen Verfahrens dienen kann, dergestalt, daß man den cyclischen Polyether in dem Polyisocyanat bzw. dessen Lösung auflöst und das Alkali-Metallsalz als Feststoff unter Komplexbildung und Auflösung einrührt.

2. Der cyclische Polyether wird in einem geeigneten Lösungsmittel aufgelöst und danach das Alkali-Metallsalz unter Komplexbildung und Auflösen zudosiert. Eventuelle Trübungen werden durch Filtration entfernt.

3. Es wird wie unter 2. beschrieben verfahren, jedoch unter Verwendung eines relativ leichtflüchtigen Lösungsmittels, welches im Anschluß an die Komplexbildung abgezogen wird, so daß der Komplex als fester Rückstand anfällt, der anschließend in einem

Le A 20 780

anderen Lösungsmittel und/oder in dem zu trimerisierenden Polyisocyanat gelöst wird.

Bei der Herstellung der 1:1-Komplexe werden vorzugsweise die Komponenten (i) und (ii) in äquimolaren Mengen eingesetzt. Selbstverständlich wäre es auch möglich, in anderen Mengenverhältnissen zu arbeiten, was jedoch zur Folge hätte, daß entweder die basische Alkali-Metallverbindung oder der cyclische Polyether im Überschuß vorliegt. Wie leicht einzusehen ist, wäre eine derartige Verfahrensweise wenig zweckmäßig, da der jeweilige Überschuß keine oder nur eine vergleichsweise schlechte katalytische Wirkung aufweisen würde. Bei der Herstellung von Lösungen der 1:1-Komplexe werden die Komponenten (i) und (ii) im allgemeinen in solchen Mengen eingesetzt, daß 0,4 bis 40, vorzugsweise 0,8 bis 20 gew.-%ige Lösungen der Komplexe vorliegen. Es ist gerade einer der Hauptvorteile der erfindungswesentlichen Katalysatoren, daß sie in derartigen, vergleichsweise hohen Konzentrationen in den nachstehend beispielhaft genannten Lösungsmitteln löslich sind.

Lösungsmittel, die, wie oben aufgezeigt, gegebenenfalls auch als Reaktionsmedium zur Herstellung der Komplexe verwendet werden können, sind insbesondere die in der Polyurethan-Lack-Technologie üblichen polaren, physiologisch weitgehend unbedenklichen Lösungsmittel eines bei Normaldruck zwischen 50°C und 350°C liegenden Siedepunkts oder alkoholische Hydroxylgruppen aufweisende, bei Raumtemperatur flüssige Verbindungen eines zwischen

Le A 20 780

32 und 250, vorzugsweise 46 und 162 liegenden Molekulargewichts. Selbstverständlich können auch beliebige Gemische derartiger Lösungsmittel eingesetzt werden.
Beispiele geeigneter Lacklösungsmittel der genannten
Art sind: Ethylacetat, Butylacetat, Ethylglykolacetat,
Aceton, Methylethylketon, Methylisobutylketon, Cyclohexanon, Methoxyhexanon oder auch Chlorkohlenwasserstoffe wie z.B. Chloroform oder Chlorbenzol. Mit
Verschnittmitteln wie z.B. Toluol, Xylol und höheren
Aromaten besteht nur eine begrenzte Löslichkeit. Höhere
Zusätze derartiger Lösungsmittel können zu Trübungen
und Ausfällungen führen. Beispiele von als Lösungsmittel
geeigneten Verbindungen mit alkoholischen Hydroxylgruppen
sind: Methanol, Ethanol, Isopropanol, Ethylenglykolacetat,
Ethylenglykol, Diethylenglykol, Ethylenglykol-monoethylether, Glycerin oder Trimethylolpropan. Da die 1:1-
Komplexe in derartigen, Hydroxylgruppen aufweisenden
Verbindungen im allgemeinen eine sehr gute Löslichkeit
aufweisen, so daß die alkoholischen Lösungsmittel nur
in sehr geringen Mengen eingesetzt werden müssen, stört
ihre Anwesenheit bei der Durchführung des erfindungsgemäßen Verfahrens nicht.

Neben diesen genannten Lösungsmitteln können auch höher
siedende Lösungsmittel wie z.B. die üblichen Weichmacher
wie Dibutylphthalat, Butylbenzylphthalat oder Phosphorsäureester wie Tricresylphosphat Verwendung finden.

Zur Durchführung des erfindungsgemäßen Verfahrens geeignete Polyisocyanate sind im Prinzip alle beliebigen

Le A 20 780

organischen Polyisocyanate mit aliphatisch, cycloaliphatisch, araliphatisch, heterocyclisch und/oder
aromatisch gebundenen Isocyanatgruppen des Molekulargewichtsbereichs 140-300 wie z.B. Tetramethylendiisocyanat,
Hexamethylendiisocyanat, 1-Isocyanato-3,3,5-trimethyl-
5-isocyanato-methyl-cyclohexan (Isophorondiisocyanat,
abgekürzt: IPDI), die isomeren Xylylendiisocyanate,
2,4- und 2,6-Diisocyanato-toluol, oder 2,4- bzw. 4,4'-
Diisocyanato-diphenylmethan, sowie beliebige Gemische
derartiger Polyisocyanate. Zu den bevorzugten Ausgangsmaterialien für das erfindungsgemäße Verfahren gehören
2,4-Diisocyanato-toluol, 2,6-Diisocyanato-toluol, beliebige Gemische dieser Isomeren, Hexamethylendiisocyanat,
IPDI, sowie beliebige Gemische dieser bevorzugten
Diisocyanate. Gut geeignet sind insbesondere auch Abmischungen der letztgenannten Diisocyanate mit aliphatisch
gebundenen Isocyanatgruppen mit aromatischen Diisocyanaten der letztgenannten Art im Gewichtsverhältnis 1:3 bis
3:1.

Grundsätzlich können beim erfindungsgemäßen Verfahren
als Ausgangsmaterialien auch Isocyanatgruppen aufweisende
NCO-Prepolymere, wie sie durch Umsetzung überschüssiger
Mengen der oben beispielshaft genannten Diisocyanate
mit Verbindungen mit gegenüber Isocyanatgruppen reaktionsfähigen Gruppen erhalten werden oder auch höher
funktionelle Polyisocyanate beispielsweise Polyisocyanatgemische wie sie bei der Phosgenierung von Anilin/
Formaldehyd-Kondensaten entstehen, eingesetzt werden,
obwohl die Verwendung derartiger modifizierter bzw.

Le A 20 780

- 12 -

höherfunktioneller Polyisocyanate weniger bevorzugt ist. Grundsätzlich können beim erfindungsgemäßen Verfahren auch Gemische aus Diisocyanaten und Monoisocyanaten als Ausgangsmaterialien eingesetzt werden, um so zu interessanten Isocyanuratgruppen aufweisenden Polyisocyanaten mit einer gezielt erniedrigten NCO-Funktionalität zu gelangen. Hierbei werden die Di- und Monoisocyanate im allgemeinen in einem Molverhältnis Diisocyanat:Monoisocyanat von 1,5:1 bis 2,5:1 eingesetzt. Geeignete Monoisocyanate sind beispielsweise aliphatische Monoisocyanate mit 1 bis 18, vorzugsweise 4 bis 8 Kohlenstoffatomen wie Methylisocyanat, n-Butylisocyanat, n-Octylisocyanat oder Stearylisocyanat oder aromatische Monoisocyanate wie insbesondere Phenylisocyanat.

Bei der Durchführung des erfindungsgemäßen Verfahrens wird lediglich ein Teil der Isocyanatgruppen des Ausgangspolyisocyanats trimerisiert. Dies bedeutet, daß die Trimerisierungsreaktion bei einem Trimerisierungsgrad (Trimerisierungsgrad = Prozentsatz der trimerisierten Isocyanatgruppen, bezogen auf Gesamtzahl der ursprünglich vorliegenden Isocyanatgruppen) von 10 bis 70 % abgebrochen wird. Falls das erfindungsgemäße Verfahren in Anwesenheit von Lösungsmitteln durchgeführt wird, so daß direkt Lösungen der erfindungsgemäßen Verfahrensprodukte entstehen, die beispielsweise als solche als Polyisocyanatkomponente in Lacken eingesetzt werden, wird die Trimerisierungsreaktion vorzugsweise bei einem Trimerisierungsgrad zwischen 50 und 70 % abgebrochen, um den Gehalt der Lösungen

Le A 20 780

an nicht-umgesetztem Ausgangsisocyanat möglichst niedrig zu halten. Falls in Abwesenheit von Lösungsmitteln gearbeitet wird, insbesondere falls das nicht-umgesetzte Ausgangsdiisocyanat nach Abbruch der Trimerisierungsreaktion, beispielsweise durch Dünnschichtdestillation, entfernt werden soll, wird die Trimerisierungsreaktion im allgemeinen bei einem Trimerisierungsgrad von 10 bis 50 %, vorzugsweise 20 bis 40 %, abgebrochen.

Geeignete Katalysatorengifte sind beispielsweise Säurechloride wie Benzoylchlorid, Acetylchlorid, Oxalylchlorid, Bernsteinsäuredichlorid, Terephthalsäuredichlorid, 2,5-Dichlorbenzylsäurechlorid, Phosphortrichlorid oder Thionylchlorid bzw. starke Säuren wie p-Toluolsulfonsäure, Nonafluorbutansulfonsäure oder Phosphorsäure sowie Carbaminsäurechloride, wie sie durch Addition von HCl an Isocyanate gebildet werden können, die unter Neutralisation der basischen Alkalimetallverbindungen die erfindungswesentlichen Katalysatoren desaktivieren.

Die Herstellung der Isocyanatgruppen aufweisenden Polyisocyanate kann in Substanz oder in Gegenwart von geeigneten Lösungsmitteln erfolgen. Geeignete Lösungsmittel sind insbesondere die oben bereits beispielhaft genannten Lacklösungsmittel, die keine gegenüber Isocyanatgruppen reaktionsfähigen Gruppen aufweisen.

Das erfindungsgemäße Verfahren wird im allgemeinen im Temperaturbereich von 0 bis 80°C, vorzugsweise 20 bis 60°C durchgeführt. Die einzusetzende Katalysatormenge hängt von der Natur des Ausgangspolyisocyanats ab und

Le A 20 780

liegt im allgemeinen bei 0,001 bis 1,0 Gew.-%, vorzugsweise 0,003 bis 0,5 Gew.-%, bezogen auf das Gewicht des 1:1-Komplexes einerseits und das Gewicht des Ausgangspolyisocyanats andererseits, wobei bei aromatischen Ausgangspolyisocyanaten der besonders bevorzugte Bereich 0,003 bis 0,05 Gew.-% und bei aliphatischen Ausgangspolyisocyanaten 0,03 bis 0,5 Gew.-% beträgt.

Die Zudosierung des Katalysators kann ebenso wie die oben bereits geschilderte Herstellung des Katalysators nach verschiedenen Methoden erfolgen:

1.  Bei der bereits oben unter 1. genannten Herstellung des Katalysators im Ausgangspolyisocyanat springt die Trimerisierungsreaktion nach Bildung des Komplexes spontan an. Bei dieser Variante werden somit die einzelnen Komponenten des Komplexes dem Ausgangspolyisocyanat getrennt zudosiert.

2.  Der Katalysator kann dem Ausgangspolyisocyanat prinzipiell auch in fester Form einverleibt werden.

3.  Vorzugsweise erfolgt die Zudosierung des Katalysators in Form der oben beschriebenen Lösungen in den oben beispielhaft genannten Lacklösungsmitteln und/ oder den oben beispielhaft genannten Verbindungen mit alkoholischen Hydroxylgruppen. Derartige, als Katalysatorkomponente für das erfindungsgemäße Verfahren besonders gut geeignete Lösungen der 1:1-Komplexe weisen im allgemeinen einen Feststoffgehalt von 0,4 bis 40, vorzugsweise 0,8 bis 20 Gew.-% auf.

Le A 20 780

Falls die Durchführung des erfindungsgemäßen Verfahrens in Gegenwart eines der oben beispielhaft genannten Lacklösungsmittel erfolgt, kann es oft zweckmäßig sein, auf eine Abtrennung des Lösungsmittels zu verzichten, und die Lösung der Verfahrensprodukte in dem genannten Lösungsmittel direkt zur Herstellung von Polyurethan-Kunststoffen, beispielsweise zur Herstellung von Polyurethanlacken, einzusetzen. In einem solchen Falle wählt man vorzugsweise sowohl für den erfindungswesentlichen 1:1-Komplex als auch als Reaktionsmedium das gleiche Lösungsmittel, dessen Menge so bemessen wird, daß Lösungen der erfindungsgemäßen Verfahrensprodukte mit einem Feststoffgehalt von 20 bis 80, vorzugsweise 40 bis 60 Gew.-%, resultieren.

Nach Zugabe des Katalysators beispielsweise bei Raumtemperatur springt die Trimerisierungsreaktion sowohl im Falle der Ausgangsisocyanate mit aliphatischen als auch der mit aromatischen Isocyanatgruppen und sowohl bei Gegenwart von Lösungsmitteln als auch in deren Abwesenheit spontan an. Bei Verwendung der optimal wirkenden Menge des erfindungswesentlichen Katalysators die durch einen einfachen orientierenden Vorversuch ermittelt werden kann, steigt die Reaktionstemperatur allmählich auf 30 - 60°C an, so daß man nach einem Zeitraum von ca. 1 bis 8 Stunden infolge der schonenden Trimerisierungsreaktion ein farbloses, klares Reaktionsprodukt erhält. Nach Erreichen der Maximaltemperatur braucht der Reaktionsansatz nicht zusätzlich beheizt zu werden, jedoch kann es auch zweckmäßig sein, zur Einhaltung optimaler Reaktions-

Le A 20 780

zeiten die Reaktionstemperatur durch Kühlen oder Heizen innerhalb des Temperaturbereichs von ca. 40 - 50°C zu halten.

Wenn der jeweils gewünschte Trimerisierungsgrad erreicht ist, wird die Reaktion durch Zusatz eines der oben beispielhaft genannten Katalysatorengifte abgebrochen. Für einen vollständigen Abbruch der Trimerisierungsreaktion ist im allgemeinen ein Zusatz einer mindestens äquimolaren Menge des Katalysatorengifts, bezogen auf den 1:1-Komplex, ausreichend. Nach Beendigung der Trimerisierungsreaktion kann das Reaktionsgemisch gewünschtenfalls destillativ aufgearbeitet werden. So kann beispielsweise der Gehalt der Verfahrensprodukte an überschüssigem Ausgangspolyisocyanat durch dessen Entfernung im Dünnschichtverdampfer auf unter 3 Gew.-%, vorzugsweise unter 0,7 Gew.-% gesenkt werden.

Das erfindungsgemäße Verfahren weist gegenüber dem Stand der Technik folgende Vorteile auf:

1. Das erfindungswesentliche Katalysatorsystem eignet sich zur Trimerisierung von sowohl aromatischen als auch aliphatischen Polyisocyanaten, wobei bei niedriger Reaktionstemperatur in schwach exothermer Reaktion gearbeitet werden kann, so daß eine Beeinträchtigung der Qualität der Verfahrensprodukte durch hohe Temperaturen praktisch ausgeschlossen werden kann.

Le A 20 780

2.    Die Menge an Katalysator ist insbesondere im Falle von aromatischen Ausgangspolyisocyanaten durchweg geringer als bei den bekannten Verfahren des Standes der Technik.

3.    Die Trimerisierungsgeschwindigkeit ist so groß, daß im allgemeinen auch ohne Energiezufuhr von außen gearbeitet werden kann.

4.    Die Katalysatoren können auch im niedrigen Temperaturbereich von 20 bis 60°C spontan desaktiviert werden, wodurch Verfärbungen der Verfahrensprodukte wie sie im Falle einer thermischen Desaktivierung beobachtet werden können, ausgeschlossen bleiben.

5.    Durch die Desaktivierung des Katalysators entstehen keine anorganischen Salze, die unlöslich sind und Trübungen verursachen, wie dies normalerweise bei Verwendung von Metallverbindungen der Fall ist, die gebildeten Neutralsalze bleiben vielmehr wegen der komplexierenden Wirkung der Kronenether gelöst, so daß klare, farblose Endprodukte entstehen.

6.    Die erfindungsgemäßen Verfahrensprodukte sind lagerstabil, Nebenprodukte wie Uretdione oder Carbodiimide werden nicht gebildet.

Die erfindungsgemäßen Verfahrensprodukte stellen wertvolle Rohstoffe zur Herstellung von Polyurethanen nach dem Isocyanat-Polyadditionsverfahren dar. Sie eignen

Le A 20 780

sich insbesondere als Isocyanatkomponente in Zweikomponenten-Polyurethanlacken. Hierzu können sie auch
in mit Blockierungsmitteln für Isocyanatgruppen blockierter Form eingesetzt werden. Ein weiteres wichtiges
Einsatzgebiet für die erfindungsgemäßen Verfahrensprodukte ist deren Verwendung als Vernetzer für Klebstoffe auf Basis von hochmolekularen Verbindungen mit
gegenüber Isocyanatgruppen reaktionsfähigen Wasserstoffatomen.

Die in den nachfolgenden Beispielen genannten Prozentangaben beziehen sich auf Gewichtsprozente.

Le A 20 780

Beispiel 1

100 g eines Gemisches aus 65 Gew.-% 2,4- und 35 Gew.-% 2,6-Toluylendiisocyanat werden bei 40°C mit 0,05 ml einer 0,2 molaren Lösung von Kaliumacetat/18-Krone-6 in Diethylenglykolmonomethylether (0,0036 % Gesamtkatalysatorkonzentration) versetzt. Die Trimerisierung setzt unter Erwärmung sofort ein, wobei eine Maximaltemperatur von 56°C erreicht wird. Nach 15 Minuten Rühren beträgt der NCO-Gehalt 38,8 %. Die Reaktion wird durch Zugabe von 0,05 ml einer 0,2 molaren Lösung von Benzoylchlorid in Diethylenglykoldimethylether abgebrochen und 30 Minuten bei 40°C nachgerührt.

Beispiel 2

100 g 2,4-Toluylendiisocyanat werden in 100 g wasserfreiem Butylacetat gelöst und unter Rühren bei 40°C mit 0,35 ml einer 0,1 molaren Lösung von Kaliumacetat/18-Krone-6 in 2-Ethylhexanol (0,013 % Gesamtkatalysatorkonzentration) versetzt. Die Trimerisierung setzt sofort ein, wobei die Temperatur nur minimal (42°C) ansteigt. Nach 16 Stunden 35 Minuten Rühren bei 40°C ist der NCO-Gehalt auf 7,9 % abgesunken. Die Reaktion wird durch Zugabe von 0,01 ml Nonafluorbutansulfonsäure abgebrochen und 15 Minuten bei 60°C nachgerührt. Die klare, farblose Lösung enthält 0,53 % freies Toluylendiisocyanat (ber. auf Feststoff) und besitzt eine Viskosität (25°C): 3054 mPas.

Le A 20 780

Beispiel 3

100 g 2,4-Toluylendiisocyanat werden in 100 g wasserfreiem Butylacetat gelöst und unter Rühren bei Zimmertemperatur mit 5 ml einer 0,01 molaren Lösung von Kaliumacetat/18-Krone-6 in Butylacetat (0,018 % Gesamtkatalysatorkonzentration) versetzt. Die Trimerisierung setzt unter Erwärmung sofort ein, wobei eine Maximaltemperatur von 57°C erreicht wird. Nach 8 Stdn. 20 Min. Rühren ohne zusätzliches Heizen ist der NCO-Gehalt auf 8,9 % abgesunken. Die Reaktion wird durch Zugabe von 0,007 g Benzoylchlorid abgebrochen und 10 Min. nachgerührt. Die klare, farblose Lösung enthält 0,43 % freies Toluylendiisocyanat (ber. auf 100 %ige Ware) und besitzt eine Viskosität (25°C): 2390 mPas.

Beispiel 4

1044 g (6 Mol) 2,4-Toluylendiisocyanat werden in 1000 g wasserfreiem Butylacetat gelöst und unter Rühren bei Zimmertemperatur mit 60 ml einer 0,01 molaren Lösung von Kaliumacetat/18-Krone-6 in Butylacetat (0,021 % Gesamtkatalysatorkonzentration) versetzt. Die Trimerisierung setzt unter Erwärmung sofort ein, wobei nach 2 Stdn. eine Maximaltemperatur von 60°C erreicht wird. Nach weiteren 3 Stdn. Rühren ohne zusätzliches Heizen beträgt der NCO-Gehalt 9,0 %. Die viskose Lösung wird nun in 10 Proben zu ca. 208 g aufgeteilt und diese werden mit jeweils 0,06 mMol Benzoylchlorid, Phosphor-

Le A 20 780

säure, Thionylchlorid, Phosphortrichlorid, Acetylchlorid, Oxalylchlorid, Bernsteinsäuredichlorid,
Terephthalsäuredichlorid, 2,5-Dichlorbenzoesäurechlorid
abgestoppt (Probe 10 ohne Stopper). Nach 2 Monaten
beträgt der NCO-Gehalt aller 9 Proben 9,0 %. Bei der
Probe 10 (ohne Stopper) ist der NCO-Gehalt im gleichen
Zeitraum auf den Wert von 7,3 % NCO abgesunken.

Beispiel 5

100 g eines Gemisches aus 65 Gew.-% 2,4- und 35 Gew.-%
2,6-Toluylendiisocyanat werden bei Zimmertemperatur mit
3 ml einer 0,01 molaren Lösung von Kaliumacetat/
18-Krone-6 in Butylacetat (0,015 % Gesamtkatalysatorkonzentration) versetzt. Die Trimerisierung setzt unter
Erwärmung sofort ein, wobei eine Maximaltemperatur von
79°C erreicht wird. Nach 25 Min. Rühren beträgt der
NCO-Gehalt 32,7 %. Die Reaktion wird durch Zugabe von
0,0035 ml Benzoylchlorid abgebrochen und 10 Minuten
nachgerührt.

Beispiel 6

504 g (3 Mol) Hexamethylendiisocyanat werden unter
Rühren bei Zimmertemperatur mit 0,46 g (0,091 %) eines
vorformulierten, kristallinen Komplexes aus Kaliumacetat
und 18-Krone-6 versetzt. Die schwach exotherme Trimerisierung (Maximaltemperatur 37°C) setzt sofort ein.
Nach 3 Stdn. 35 Min. beträgt der NCO-Gehalt 42 %. Die
Reaktion wird durch Zugabe von 0,15 ml Benzoylchlorid abgebrochen und 15 Min. nachgerührt. Die schwach gelblich gefärbte Lösung wird filtriert (ein Teil des Komplexes bleibt unlöslich im HDI). Nach Dünnschichtdestillation erhält man 125 g eines leicht gelblichen Produktes. NCO-Gehalt: 22,3 %, Viskosität (25°C): 1949 mPas, Gehalt an monomerem HDI:<0,1 %.

Aus der Gelchromatographie ergeben sich 63 % Monosiocyanurat und 35 % höhermolekulare Polyisocyanate mit Isocyanuratstruktur.

Beispiel 7

504 g (3 Mol) Hexamethylendiisocyanat werden unter Rühren bei Zimmertemperatur mit 1,53 ml einer 0,5 molaren Lösung von Kaliumacetat/18-Krone-6 in Diethylenglykolmonomethylether (0,055 % Gesamtkatalysatorkonzentration) versetzt. Die exotherme Trimerisierung (Maximaltemperatur 58°C) setzt sofort ein. Nach 45 Min. beträgt der NCO-Gehalt der Lösung 40,8 %. Die Reaktion wird durch Zugabe von 5 mg Nonafluorbutansulfonsäure abgebrochen und 15 Min. nachgerührt. Nach Dünnschichtdestillation ohne vorherige Filtration erhält man 140 g eines klaren, fast farblosen Produktes, NCO-Gehalt: 23 %, Viskosität (25°C): 2375 mPas, Gehalt an freiem monomerem HDI: < 0,1 %.

Beispiel 8

504 g (3 Mol) Hexamethylendiisocyanat werden unter Rühren bei Zimmertemperatur mit 5 ml einer 0,1 molaren Lösung von Kaliumacetat/18-Krone-6 in 2-Ethylhexanol (0,036 % Gesamtkatalysatorsystemkonzentration) versetzt. Die

Le A 20 780

schwach exotherme Trimerisierung setzt sofort ein (Maximaltemperatur 36°C). Nach 4 Stunden 30 Minuten beträgt der NCO-Gehalt der Lösung 40,6 %. Die Reaktion wird durch Zugabe von 0,012 ml Benzoylchlorid abgebrochen und 15 Minuten nachgerührt. Dünnschichtdestillation der klaren Lösung ergibt 180 g eines klaren, leicht gelblichen Produktes, NCO-Gehalt: 22,9 %, Viskosität (25°C): 2653 mPas, Gehalt an freiem monomeren HDI: < 0,1 %.

## Patentansprüche

1. Verfahren zur Herstellung von Isocyanuratgruppen aufweisenden Polyisocyanaten durch Trimerisierung eines Teils der Isocyanatgruppen von organischen Polyisocyanaten oder von Gemischen aus Di- und Monoisocyanaten in Gegenwart von basischen Verbindungen als Trimerisierungskatalysatoren und Abbruch der Trimerisierungsreaktion durch Zugabe eines Katalysatorengifts, dadurch gekennzeichnet, daß man als Trimerisierungskatalysatoren 1:1-Komplexe von (i) basischen Natrium- oder Kaliumverbindungen und (ii) 1,4,7,10,13-Pentaoxacyclopentadecan bzw. 1,4,7,10,13,16-Hexaoxacyclooctadecan verwendet.

2. Verfahren gemäß Anspruch 1, dadurch gekennzeichnet, daß man als Komponente (i) ein basisches Natrium- oder Kaliumcarboxylat, -alkoholat, -phenolat, -carbonat, -hydroxid, -cyanat oder -cyanid verwendet.

3. Verfahren gemäß Anspruch 1 und 2, dadurch gekennzeichnet, daß man als 1:1-Komplex einen Komplex aus Kaliumacetat und 1,4,7,10,13,16-Hexaoxacyclooctadecan verwendet.

4. Verfahren gemäß Anspruch 1 bis 3, dadurch gekennzeichnet, daß man den 1:1-Komplex in Form einer Lösung in einem polaren Lacklösungsmittel und/oder mindestens einer bei Raumtemperatur flüssigen,

Le A 20 780

0056158

alkoholische Hydroxylgruppen aufweisenden Verbindung des Molekulargewichtsbereichs 32 - 250 verwendet.

5. Als Katalysatorkomponente zur Durchführung des Verfahrens gemäß Anspruch 1 bis 4 geeignete Lösungen von 1:1-Komplexen von (i) basischen Natrium- oder Kaliumverbindungen und (ii) 1,4,7,10,13-Pentaoxacyclopentadecan bzw. 1,4,7,10,13,16-Hexaoxacyclooctadecan in polaren Lacklösungsmitteln und/oder mindestens einer bei Raumtemperatur flüssigen, alkoholische Hydroxylgruppen aufweisenden Verbindung des Molekulargewichtsbereichs 32 - 250.

6. Verwendung der gemäß Anspruch 1 bis 4 erhältlichen Isocyanatgruppen aufweisenden Polyisocyanate, gegebenenfalls in von monomerem Ausgangspolyisocyanat befreiter Form und/oder gegebenenfalls in mit Blockierungsmitteln für Isocyanatgruppen blockierter Form als Isocyanatkomponente bei der Herstellung von Polyurethanen.

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 622 577 (PEDERSEN) <br> * Ansprüche 1, 3; Spalte 5, Zeilen 66 bis 67; Spalte 6, Zeilen 69 bis 75; Spalte 7, Zeilen 6 bis 21, 32 bis 35, 39 bis 46 * <br> -- | 1,2,6 |
| X | GB - A - 1 285 367 (DU PONT) <br> * Ansprüche 8, 10, 13; Seite 4, Zeilen 39 bis 41, 62 bis 92; Seite 5, Zeilen 61 bis 67 * <br> & DE - A - 1 963 528 <br> -- | 5 |
| A | Patent Abstracts of Japan <br> Band 2, Nr. 122, 13. Oktober 1978 <br> Seite 2441C78 <br> & JP - A - 53 - 87379 <br> -- | |
| A | DE - B2 - 1 954 093 (MOBAY CHEMICAL) <br> * Patentanspruch * <br> -- | 1 |
| A | US - A - 2 952 665 (BUNGE et al.) <br> * Anspruch 6; Spalte 3, Zeilen 66 bis 70 * <br> ---- | 6 |

### KLASSIFIKATION DER ANMELDUNG (Int. Cl.)

C 08 G 18/02
C 07 D 251/34
C 08 G 18/22
C 07 D 323/00
C 08 G 18/79
C 08 G 18/80

### RECHERCHIERTE SACHGEBIETE (Int. Cl.)

C 07 D 251/00
C 07 D 323/00
C 08 G 18/00

### KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| | Der vorliegende Recherchenbericht wurde für alle Patentansprüche erstellt. |
|---|---|

| Recherchenort | Abschlußdatum der Recherche | Prüfer |
|---|---|---|
| Berlin | 07-04-1982 | MARX |

EPA form 1503.1 06.78